# EUROPÄISCHE PATENTSCHRIFT

(11) **EP 1 590 351 B1**
(45) Veröffentlichungstag und Bekanntmachung des Hinweises auf die Patenterteilung: **03.09.2008**
(21) Anmeldenummer: 04704202.3
(22) Anmeldetag: 22.01.2004
(51) Int. Cl.: C07D 487/04

(54) **VERFAHREN ZUR HERSTELLUNG VON CHINAZOLIN-ALKALOIDEN**
METHODS FOR PRODUCING QUINAZOLINE ALKALOIDS
PROCEDE DE PRODUCTION D'ALCALOIDES DU TYPE QUINAZOLINE

(30) Priorität: 03.02.2003 DE 10304141
(43) Veröffentlichungstag der Anmeldung: 02.11.2005
(73) Patentinhaber: HF ARZNEIMITTELFORSCHUNG GmbH, 59368 Werne (DE)
(72) Erfinder: MOORMANN, Joachim, 59368 Werne (DE); HOFFMANN, Hans-Rainer, 56566 Neuwied (DE); MATUSCH, Rudolf, 35041 Marburg (DE)
(74) Vertreter: Flaccus, Rolf-Dieter
(86) Internationale Anmeldenummer: PCT/EP2004/000485
(87) Internationale Veröffentlichungsnummer: WO 2004/069836

(56) Entgegenhaltungen:
- JEN, T. ET AL.: "Amidines. 5. Synthesis of Pyrrolo[2,3-b]isoquinoline, Imidazo[1,2-b]isoquinoline, Pyrrolo[2,1-b]quinazoline, and 1,3-Thiazino[2,3-b]quinazoline Derivatives and Related Heterocycles as Potential Antihypertensive Agents" JOURNAL OF MEDICINAL CHEMISTRY, Bd. 16, Nr. 6, 1973, Seiten 633-637, XP002282441
- YADAV, J.S.; REDDY, B.V.S.: "Microwave-assisted rapid synthesis of the cytotoxic alkaloid luotonin A" TETRAHEDRON LETTERS, Bd. 43, 2002, Seiten 1905-1907, XP002282442
- SPÄTH, E.; PLATZER, N.: "Über Derivate des Peganins und ihre Ring-Homologen" CHEMISCHE BERICHTE, 1935, Seiten 2221-2226, XP009031343 in der Anmeldung erwähnt

## Beschreibung

Die Erfindung betrifft Verfahren zur Herstellung von Chinazolin-Alkaloiden der nachfolgenden Formeln (I) und (III).

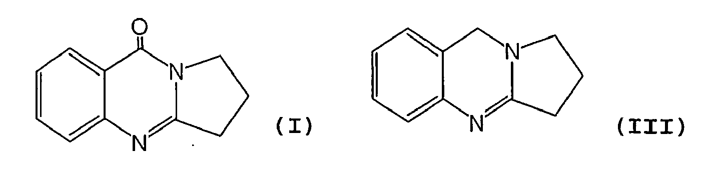

Bei Verbindung (III) handelt es sich um 1,2,3,9-Tetrahydropyrrolo[2,1-b]chinazolin. Diese Verbindung ist auch unter dem Trivialnamen Desoxypeganin bekannt und kommt in Pflanzen der Familie Zygophyllaceae vor. Aufgrund seiner pharmakologischen Eigenschaften wird Desoxypeganin zur Gruppe der reversibel wirkenden Cholinesterasehemmstoffe gezählt. Daneben wirkt es auch als Monoaminoxidase-Hemmer. Desoxypeganin wird als Arzneistoff für therapeutische Zwecke in Betracht gezogen, so z. B. zur Behandlung der Drogensucht und Drogenabhängigkeit (DE-A 199 06 978), zur Therapie der Alzheimer'schen Demenz (DE-A 199 06 975) oder zur Therapie der Nikotinabhängigkeit (DE-A 199 06 979).

Verbindung (I), unter der Bezeichnung Pegen-(9)-on-(8) bekannt, ist ein wichtiges Zwischenprodukt bei der Synthese von Desoxypeganin und anderen Chinazolin-Alkaloiden dieses Typs. Die Synthese von Pegen-(9)-on-(8) (kurz: Pegenon) kann nach SPÄTH und PLATZER (Ber. 68 (1935), 2221-2224) durch Umsetzung von Isatosäure-Anhydrid mit einer äquimolaren Menge Pyrrolidon erfolgen. Anschließend muss zur Abtrennung der Reaktionsnebenprodukte eine Hochvakuum-Destillation bei 140 bis 160 °C durchgeführt werden. Dieses Verfahren ist nur zur Herstellung von kleinen Mengen (im Gramm-Bereich) geeignet, jedoch nicht für die Produktion im größeren Maßstab.

Nach MORRIS, HANFORD und ADAMS (J. Amer. Chem. Soc. 57 (1935), 951-954) kann Pegenon (2,3-Trimethylen-4-chinazolon) durch (i) Oxidation von Desoxivasicin mittels Wasserstoffperoxid erhalten werden. Desoxivasicin ist mit Desoxypeganin identisch. Alternativ (ii) kann Pegenon durch eine mehrstufige Synthese erhalten werden, wobei γ-Phenoxybutyrylchlorid mit o-Aminobenzamid zu dem entsprechenden Amid umgesetzt wird. Nach Ringschluß unter Erhitzen wird die Phenoxylgruppe durch Brom ersetzt und anschließend durch einen weiteren Ringschluss 2,3°-Trimethylen-4-chinazolon (= Pegenon) erhalten.

Falls Pegenon als Ausgangsverbindung für die Herstellung von Desoxypeganin benötigt wird, kommt - neben der bereits erwähnten Methode von SPÄTH und PLATZER - allenfalls der von MORRIS et al. vorgeschlagene Syntheseweg (ii) in Betracht. Dieser ist allerdings wegen der Anzahl der Reaktionsschritte unwirtschaftlich.

Die Gewinnung von Desoxypeganin erfolgt vorzugsweise durch Isolierung aus der Steppenraute (Peganum harmala) oder durch Synthese.

MORRIS, HANFORD und ADAMS (a.a.O., S. 953) beschreiben die Herstellung von Desoxyvasicin (= Desoxypeganin) durch Reduktion von 2,3-(α-Hydroxytrimethylen)-4-chinazolon oder 2,3-(α-Chlorotrimethylen)-4-chinazolon unter Verwendung von Eisessig und Zinkstaub (Reaktionstyp: Clemmensen-Reduktion). Nach Chloroform-Extraktion wurde das Produkt durch Kristallisation aus Petroleumether isoliert. Nachteilig ist hierbei, dass die Ausgangsverbindungen (2,3-(α-Hydroxytri-methylen)-4-chinazolon oder 2,3-(α-Chlorotrimethylen)-4-chinazolon) ausgehend von Peganin (= Vasicin) synthetisiert werden müssen.

Nach SARGAZAKOV et al. (Khim. Prir. Soedin. 4 (1990), 506-507) kann Desoxypeganin-Hydrochlorid durch Cyclokondensation von Anthranilsäure mit 2-Pyrrolidon zu Desoxyvasicinon-Hydrochlorid (= Pegenon-Hydrochlorid) und nachfolgende Clemmensen-Reduktion dieses Zwischenprodukts zu Desoxypeganin-Hydrochlorid erhalten werden. Die Cyclokondensationsreaktion wird in Gegenwart von Phosphortrichlorid durchgeführt, wobei Toluol als Lösemittel verwendet wird. Sowohl nach dem ersten Schritt (Cyclokondensation) als auch nach dem zweiten Schritt (Reduktion) sind mehrfache Chloroform-Extraktionen erforderlich. Insgesamt werden für die Herstellung von 2 kg Desoxypeganin-Hydrochlorid mindestens ca. 50 1 Toluol und 80 1 Chloroform benötigt.

Das von SARGAZAKOV et al. beschriebene Syntheseverfahren ist aus mehreren Gründen nachteilig. Die Ausbeute beträgt lediglich ca. 50 %, wobei das erhaltene Produkt (Desoxypeganin-Hydrochlorid) eine Reinheit von 94-95 % aufweist. Bei diesem Verfahren werden größere Mengen an organischen Lösungsmitteln, insbesondere Toluol und Chloroform, benötigt, sowie Phosphortrichlorid. Dies ist aus ökologischen Gründen, aber auch aus Sicherheits- und Kostengründen nachteilig. Das Verfahren ist zudem sehr zeit- und arbeitsaufwendig, da mehrere Chloroform-Extraktionsschritte durchgeführt werden müssen, um den genannten Reinheitsgrad zu erzielen. Im Hinblick auf die Verwendung von Desoxypeganin als Arzneistoff ist insbesondere die Verwendung chlorierter Kohlenwasserstoffe besonders nachteilig.

Ferner ist bei den erwähnten bekannten Syntheseverfahren von Nachteil, dass aufgrund des hohen Anteils von Reaktionsnebenprodukten eine Isolation der Produkte durch Kristallisationsverfahren nicht möglich ist, sondern nur durch aufwendige Extraktionsschritte oder Hochvakuumdestillationen erreicht werden kann.

Der hier beschriebenen Erfindung lag deshalb die Aufgabe zugrunde, Syntheseverfahren für die Herstellung von Chinazolin-Alkaloiden der oben genannten Formeln (I) und (III) anzugeben, wobei diese Verfahren
- Ausbeuten und Produktreinheitsgrade ermöglichen, die denjenigen bekannter Verfahren zumindest entsprechen oder diese übertreffen;
- die Herstellung von Desoxypeganin (III) für die Verwendung zur Herstellung von Medikamenten ermöglichen;
- weitgehend auf den Einsatz umwelt- oder gesundheitsgefährdender Stoffe, insbesondere organischer Lösemittel, verzichten;
- eine gute Rohstoffbilanz aufweisen;
- im wesentlichen nur solche Nebenprodukte erzeugen, die gut rezyklisierbar sind;
- mit kostengünstigen Ausgangsmaterialien durchgeführt werden können;
- insgesamt eine einfachere und kostengünstigere Herstellung der genannten Verbindungen im großtechnischen Maßstab ermöglichen.

Diese und andere Aufgaben werden überraschenderweise durch Verfahren gemäß den unabhängigen Ansprüchen 1, 9, 18 und 20 gelöst, sowie durch die in den abhängigen Ansprüchen beschriebenen weiteren Ausführungsformen.

Nach Anspruch 1 wird bei einem erfindungsgemäßen Verfahren eine Verbindung der Formel (II) (= Isatosäureanhydrid) 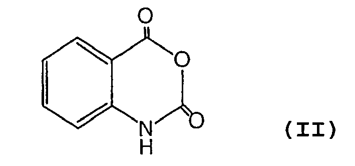 mit 2-Pyrrolidon (= Pyrrolidinon) umgesetzt, um eine Verbindung der Formel (I) zu erhalten. Dabei wird 2-Pyrrolidon im Überschuss eingesetzt, bezogen auf die eingesetzte Menge an Verbindung (II). Das heißt, die Menge des eingesetzten Pyrrolidons ist größer als die äquimolare Menge. Bevorzugt werden 1,5 bis 5 Mol, insbesondere 2 bis 4 Mol, und am meisten bevorzugt 2,5 bis 3,5 Mol, Pyrrolidon eingesetzt, wobei sich diese Angaben jeweils auf die eingesetzte Menge an Verbindung (II) beziehen.

Dies hat überraschenderweise zur Folge, dass die Bildung ungünstiger Reaktionsnebenprodukte vermindert wird und das entstehende Produkt (I) gut kristallisierbar ist. Zudem können hohe Ausbeuten (70 %) erzielt werden, wobei die Reinheit ca. 99 % beträgt (NMR). Dank der ausgezeichneten Kristallisierbarkeit kann auf aufwendige Reinigungsmethoden, wie Hochvakuumdestillation, verzichtet werden.
Da die Reinigung des Produkts auf einfache Weise durch Kristallisation erreicht werden kann, lässt sich das Verfahren auch problemlos im größeren Maßstab durchführen.

Dem Verfahren liegt folgende beispielhafte Reaktionsgleichung zugrunde:

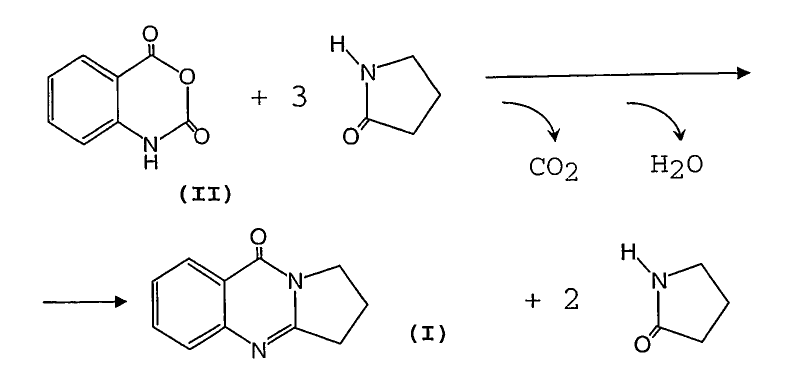

Die Reaktion wird unter Wärmezufuhr durchgeführt, vorzugsweise bei Temperaturen im Bereich von 50 bis 200 °C. Dabei wird bevorzugt, dass anfangs eine Temperatur im Bereich von 70 bis 130 °C und nachfolgend im Bereich von 140 bis 200 °C eingehalten wird; besonders bevorzugt beträgt die Temperatur anfangs 80 bis 120 °C und nachfolgend 150 bis 190 °C.

Die genannte anfängliche Temperatur wird vorzugsweise über einen Zeitraum von 0,5 bis 2 h, insbesondere 1 bis 1,5 h, nach Start der Reaktion eingehalten. Die genannte nachfolgende Temperatur wird vorzugsweise über einen Zeitraum von 1 bis 8 h, insbesondere 2 bis 5 h, eingehalten.

Das Verfahren wird bevorzugt in der Weise durchgeführt, dass Pyrrolidon vorgelegt und danach Verbindung (II) portionsweise dazugegeben wird.

Besonders vorteilhaft ist es, das Produkt (I) direkt aus dem Reaktionsgemisch durch Kristallisation zu isolieren. Zur Kristallisation der Verbindung (I) wird der Reaktionsansatz abkühlen gelassen und mit Impfkristallen angeimpft. Die Kristallisation wird bei Raumtemperatur, vorzugsweise bei mindestens 25 °C, bevorzugt bei 30 bis 70 °C, am meisten bevorzugt bei 40 bis 60 °C, durchgeführt, wodurch der Verlauf der Kristallisation beschleunigt wird. Der Kristallisationsvorgang dauert ca. 24 h bis 7 Tage, bevorzugt ca. 50 h bis 100 h. Die sich bildenden Kristalle weisen einen Gehalt an Pyrrolidon auf.

Die vorliegende Erfindung betrifft ferner Verfahren zur Herstellung von Desoxypeganin (Verbindung gemäß Formel III). Die Verfahren umfassen im wesentlichen drei Schritte:
(A) Herstellung von Verbindung (I) nach einem Verfahren gemäß einem der Ansprüche 1 bis 8;
(B) Reduktionsreaktion, wobei Verbindung (III) in Salzform erhalten wird;
(C) Freisetzung von Verbindung (III) aus dem Salz.

Der Verlauf der Reduktionsreaktion (Clemmensen-Reduktion) lässt sich beispielhaft wie folgt darstellen:

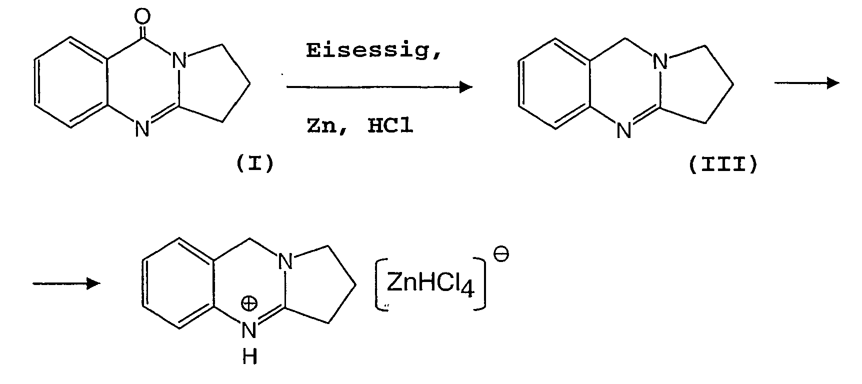

Verbindung (III) wird dabei in Form eines Salzes erhalten; im oben beispielhaft dargestellten Fall erhält man bei Durchführung der Reduktionsreaktion in Gegenwart von Eisessig, Zink und Salzsäure ein Tetrachlorozinkatsalz.

Als Ausgangsverbindung (I) wird besonders bevorzugt ein durch Kristallisation isoliertes Reaktionsprodukt verwendet, das durch die oben beschriebenen Verfahren erhalten werden kann.

Nach einer bevorzugten Ausführungsform der Erfindung wird die Reduktionsreaktion unter Verwendung von Eisessig, Zink (Zinkstaub) und Salzsäure durchgeführt, wobei vorzugsweise so vorgegangen wird, dass Verbindung (I) zunächst in Eisessig gelöst wird und nachfolgend Zink und Salzsäure dazugegeben werden.

Nach einer weiteren bevorzugten Ausführungsform wird die Reduktionsreaktion unter Zusatz von Zink (als Zinkstaub) und Schwefelsäure durchgeführt, ohne Verwendung von Eisessig. Man erhält das Hydrogensulfat von Verbindung (III):

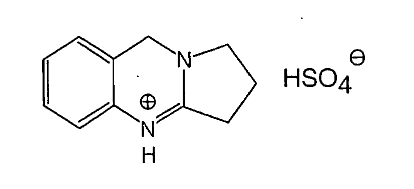

Vorzugsweise wird die Reaktion bei Temperaturen von ca. 50 bis 90 °C, insbesondere 70 bis 80 °C, durchgeführt; im allgemeinen ist die Reduktion nach ca. 2 bis 3 h quantitativ erfolgt. Nach Beendigung der Reaktion wird das überschüssige Zink durch bekannte Methoden, z. B. durch Kolieren, entfernt.

Das so erhaltene Salz der Verbindung (III) kann auf einfache und effiziente Weise durch Kristallisation aus dem Reaktionsgemisch isoliert werden. Hierzu wird der Reaktionsansatz mit Impfkristallen angeimpft. Nach erfolgter Kristallisation wird die verbleibende Mutterlauge durch dem Fachmann bekannte Methoden abgetrennt (z. B. Dekantieren).

Im genannten Schritt (C) der erfindungsgemäßen Verfahren zur Herstellung einer Verbindung der Formel (III) wird sodann das in Schritt (B) erhaltene Salz in die freie Base (III) überführt.

Dies erfolgt allgemein durch Zusatz einer Base, vorzugsweise NaOH, zu einer wässrigen Lösung des Salzes.

Gemäß einer besonders bevorzugten Ausführungsform wird dieser Schritt in der Weise durchgeführt, dass die freigesetzte Base in geschmolzener Form erhalten wird. Dies wird dadurch erreicht, dass die Reaktion bei einer Temperatur durchgeführt wird, die höher ist als der Schmelzpunkt (86 °C) der freien Base (III) (=Desoxypeganin), vorzugsweise bei Temperaturen im Bereich von 90 bis 100 °C. Die in geschmolzener Form vorliegende Base kann auf einfache Weise, gegebenenfalls nach dem Erstarren, aus dem Reaktionsansatz abgetrennt werden; hierfür geeignete Methoden sind dem Fachmann bekannt.

Nach einer weiteren besonders bevorzugten Ausführungsform wird die geschmolzene Base (III) nachfolgend abgekühlt und erstarren gelassen. Das erstarrte Produkt wird in Lösung gebracht, um eine gesättigte Lösung zu erhalten; vorzugsweise wird Wasser als Lösemittel verwendet. Aus dieser gesättigten Lösung, die durch Basenzusatz (vorzugsweise NaOH) alkalisch eingestellt wird, kann durch den sich anschließenden Kristallisationsvorgang das Endprodukt Desoxypeganin in hochreiner Form isoliert werden.

Die Erfindung erstreckt sich somit auf Verfahren zur Herstellung von Desoxypeganin (Verbindung der Formel (III), welche einen Schritt aufweisen, bei welchem diese Verbindung in flüssiger Form aus dem Reaktionsansatz abgeschieden wird. Die Erfindung schließt insbesondere Herstellungsverfahren der genannten Art mit ein, die folgende Verfahrensschritte aufweisen:
- Reduktion der genannten Verbindung (I) zu Verbindung (III), wobei Verbindung (III) in Salzform anfällt;
- Zusatz einer Base, wodurch Verbindung (III) aus dem Salz freigesetzt und in flüssiger Form abgeschieden wird.

Die erfindungsgemäßen Verfahren ermöglichen eine einfache und kostengünstige Herstellung der genannten Verbindungen (I, III) in einer für die Verwendung in der Arzneimittelproduktion erforderlichen Menge und Reinheit. Besonders vorteilhaft ist dabei, dass auf den Einsatz von organischen Lösemitteln verzichtet werden kann.

Die erfindungsgemäßen Verfahren werden nachfolgend anhand von Beispielen näher erläutert.

### Beispiel 1 (Vergleichsbeispiel)

Herstellung von Pegen-9-(on)-(8) (identisch mit Formel (I)) durch Umsetzung von Pyrrolidon mit einer äquimolaren Menge Isatosäure-Anhydrid nach SPÄTH und PLATZER (a.a.O., S. 2221 u. 2224).

Zwei Liter Pyrrolidon wurden mit 4 kg Isatosäureanhydrid (molares Mengenverhältnis 1:1,07) in einem Chromstahlbehälter (Fassungsvermögen 10 l) unter kräftigem Rühren auf 120 °C erhitzt bis zur beginnenden Gasentwicklung. Anschließend wurde 10 min auf 160 °C und schließlich 30 min auf 190 °C erhitzt. Von diesem Reaktionsansatz wurden 558 g (= 3 Mol) im noch heiß-flüssigen Zustand in einen 1-Liter-Kolben (NS 29) überführt. Der im Stahlbehälter zurückbleibende Rest erstarrte zu einer schwarzen, glasartigen Masse, die für die Isolierung von Pegenon ungeeignet ist.

Aus dem in den Kolben überführten Reaktionsansatz (558 g) konnten durch Hochvakuumdestillation über eine auf 120 °C beheizte Destillationsbrücke bis zu 117 g Pegenon destilliert werden; dies entspricht einer Ausbeute von 21 %. Nach Umkristallisieren aus Ether wurde ein Schmelzpunkt von 111 °C ermittelt.

### Beispiel 2:

### Synthese von Pegenon (Verbindung nach Formel (I))

Ein Mol Isatosäureanhydrid (Formel (II)) wurde mit 3 Mol Pyrrolidon umgesetzt. Dabei wurde Pyrrolidon in einer beheizbaren Chromstahltrommel (30 1) vorgelegt und auf ca. 100 °C erhitzt, und anschließend Isatosäureanhydrid portionsweise unter ständigem Rühren dazugegeben.
Nach ca. 1 h war die gesamte Menge Isatosäureanhydrid in Pyrrolidon gelöst. Nachfolgend wurde 5 h auf 155 bis 160 °C erhitzt (dabei Bildung von CO₂ und H₂O; s. o.) und schließlich kurzzeitig auf 170 bis 180 °C.

Die so erhaltene Masse wurde nach Abkühlen auf ca. 50 °C mit Pegenon-Kristallen angeimpft und ca. 50 bis 100 h bei Raumtemperatur kristallisieren gelassen. Durch Erhöhung der Temperatur (mindestens 25 °C) konnte der Kristallisationsvorgang beschleunigt werden (Dauer nur ca. 2 bis 3 Tage). Die "Mutterlauge" wurde durch Dekantieren von den Kristallen abgetrennt.

Insgesamt wurden mehr als 90 % der eingesetzten Ausgangsverbindungen zu Pegenon umgesetzt. Die Ausbeute an kristallisiertem Pegenon betrug 40 %; der restliche Anteil des Pegenons ist in Pyrrolidon gelöst. Die erhaltenen Pegenon-Kristalle weisen gemäß NMR noch einen Gehalt an 30 Mol-% Pyrrolidon auf; die Reinheit des Pegenons beträgt ca. 99 % nach NMR.

In einer Abwandlung dieses Verfahrens wurden die Ausgangsverbindungen in einem Mengenverhältnis von 1:2,5 (Isatosäureanhydrid:Pyrrolidon) eingesetzt. Dadurch konnte der Anteil des kristallisierten Pegenons auf ca. 55 % gesteigert werden, bei einer Reinheit von ca. 99 %.

### Beispiel 3:

### a) Clemmensen-Reduktion von Verbindung (I)

Gemäß Beispiel 2 hergestellte Pegenon-Kristalle (2,5 kg; mit einem Gehalt an 15 Gew.-% Pyrrolidon; entspricht 1,83 kg oder 9,81 Mol reinem Pegenon) wurden bei ca. 50 °C in 6,1 l (= 100,4 Mol) Eisessig gelöst und in einen Chromstahlbehälter (50 l) überführt. Anschließend wurden unter Rühren 3,7 kg (= 56,4 Mol) Zinkstaub in kleinen Portionen dazugegeben. Das Reaktionsgemisch wurde dabei auf ca. 60 °C temperiert. Nach ca. 1 h wurden 4 x 3 1 konzentrierte HCl (32 %; entspricht 121,74 Mol) innerhalb von 2 h in kleinen Portionen unter ständigem Rühren zugegeben. Danach (nach ca. 4 bis 5 h) wurde der überschüssige Zinkstaub durch Kolieren aus dem Reaktionsgemisch entfernt. Nach Animpfen mit Impfkristallen konnten ca. 66 % des Desoxypeganin-Salzes durch Auskristallisieren abgetrennt werden. Die verbleibende Mutterlauge wurde durch Verdunstung eingeengt, woraufhin wiederum 66 % des Desoxypeganin-Salzes auskristallisierten. Insgesamt konnten ca. 90 % des Desoxypeganinsalzes in kristalliner Form gewonnen werden.

### b) Freisetzung von Desoxypeganin-Base (Verbindung (III))

Auf diese Weise erhaltene Kristalle (2,5 kg) wurden in heißem Wasser (ca. 11 l) gelöst. Anschließend wurde unter Rühren NaOH zugegeben (ca. 8 kg Rotulae), wobei der Ansatz auf 95 bis 100 °C erhitzt wurde. Dabei wurde Desoxypeganin in geschmolzener Form erhalten und aus dem Reaktionsgemisch abgetrennt. Nach dem Erstarren wurde das Produkt wiederum in heißem Wasser gelöst, um eine gesättigte Lösung zu erhalten, die mittels NaOH alkalisch eingestellt wurde. Aus dieser gesättigten alkalischen Lösung wurde Desoxypeganin durch Kristallisation isoliert.

Die Ausbeute an Desoxypeganin, bezogen auf das eingesetzte Desoxypeganin-Salz, lag über 90 %, bei einer Reinheit von 99,9 % (NMR).

### Beispiel 4:

### Clemmensen-Reduktion von Verbindung (I) (alternative Methode)

175 g Pegenon-Kristalle (mit einem Gehalt an 30 Mol% Pyrrolidon; entspricht 150 g Pegenon; siehe Beispiel 2) wurden mit 750 ml H₂SO₄ (20 %ig) versetzt und ca. 30 min unter Rühren erhitzt (ca. 85 °C).
Nachfolgend wurden nach und nach 260 g Zinkstaub zugegeben (Dauer: ca. 2 h). Nach ca. 1 h wurde nochmals H₂SO₄ zugegeben (375 ml, 40 %ig). Nach insgesamt ca. 3 h war die Reaktion beendet, und das überschüssige Zink wurde durch Kolieren entfernt.

Wie unter Beispiel 3 a) beschrieben, wurde das gewonnene Desoxypeganin-Salz durch Kristallisation aus dem Reaktionsansatz abgetrennt. Die Freisetzung der Base erfolgte nach der unter Beispiel 3 b) beschriebenen Methode.

## Patentansprüche

1. Verfahren zur Herstellung einer Verbindung der nachfolgenden Formel (I) 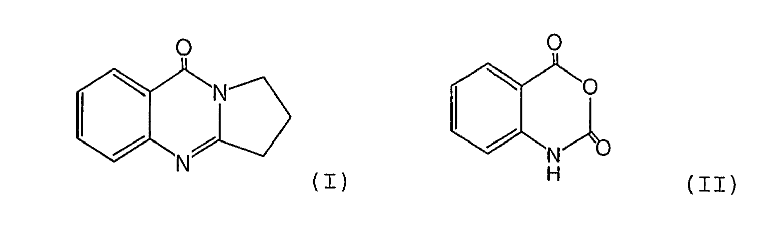 durch Umsetzung einer Verbindung der Formel (II) mit 2-Pyrrolidon, **dadurch gekennzeichnet, dass** 2-Pyrrolidon, bezogen auf Verbindung (II), im Überschuss eingesetzt wird.

2. Verfahren nach Anspruch 1, **dadurch gekennzeichnet, dass** das Reaktionsprodukt (I) in einem nachfolgenden Schritt direkt aus dem Reaktionsgemisch durch Kristallisation isoliert wird.

3. Verfahren nach Anspruch 1 oder 2, **dadurch gekennzeichnet, dass** 1,5 bis 5 Mol, vorzugsweise 2 bis 4 Mol, besonders bevorzugt 2,5 bis 3,5 Mol 2-Pyrrolidon eingesetzt werden, jeweils bezogen auf die Menge an Verbindung (II).

4. Verfahren nach einem der vorangehenden Ansprüche, **dadurch gekennzeichnet, dass** die Umsetzung bei 50 bis 200 °C durchgeführt wird.

5. Verfahren nach Anspruch 4, **dadurch gekennzeichnet, dass** das Reaktionsgemisch anfangs auf 70 bis 130 °C, vorzugsweise auf 80 bis 120 °C, und nachfolgend auf 140 bis 200 °C, vorzugsweise 150 bis 190 °C, temperiert wird.

6. Verfahren nach Anspruch 5, **dadurch gekennzeichnet, dass** die genannte anfängliche Temperatur über einen Zeitraum von 0,5 bis 2 h, vorzugsweise 1 bis 1,5 h, und die genannte nachfolgende Temperatur über einen Zeitraum von 1 bis 8 h, vorzugsweise von 2 bis 5 h, beibehalten wird.

7. Verfahren nach Anspruch 6, **dadurch gekennzeichnet, dass** der Reaktionsansatz nach dem Abkühlen mit Impfkristallen der Verbindung (I) angeimpft wird und über einen Zeitraum von 24 h bis 7 Tagen, vorzugsweise 50 bis 100 h, bei Raumtemperatur, vorzugsweise bei mindestens 25 °C, gehalten wird, um das Auskristallisieren zu ermöglichen.

8. Verfahren nach Anspruch 7, **dadurch gekennzeichnet, dass** die Kristallisation bei 30 bis 70 °C, vorzugsweise bei 40 bis 60 °C, durchgeführt wird.

9. Verfahren zur Herstellung einer Verbindung der nachfolgenden Formel (III), umfassend folgende Schritte: 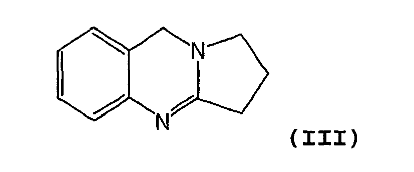
(A) Herstellung von Verbindung (I) nach einem Verfahren gemäß einem der Ansprüche 1 bis 8;
(B) Reduktionsreaktion, wobei Verbindung (III) in Salzform erhalten wird;
(C) Freisetzung von Verbindung (III) aus dem Salz.

10. Verfahren nach Anspruch 9, **dadurch gekennzeichnet, dass** als Verbindung (I) ein durch Kristallisation isoliertes Reaktionsprodukt nach einem der Ansprüche 1 bis 8 eingesetzt wird.

11. Verfahren nach Anspruch 9 oder 10, **dadurch gekennzeichnet, dass** die Reduktionsreaktion (Schritt B) in Gegenwart von Zink und Säure durchgeführt wird.

12. Verfahren nach Anspruch 11, **dadurch gekennzeichnet, dass** Verbindung (I), vorzugsweise in kristallisierter Form, zunächst in Eisessig gelöst wird und nachfolgend Zink und Salzsäure zugegeben werden.

13. Verfahren nach Anspruch 12, **dadurch gekenntzeichnet, dass** die Reduktionsreaktion in Gegenwart von wässriger Schwefelsäure und Zinkstaub durchgeführt wird.

14. Verfahren nach einem der Ansprüche 9 bis 13, **dadurch gekennzeichnet, dass** im Anschluss an Schritt (B) Verbindung (III) als Salz durch Kristallisation aus dem Reaktionsansatz isoliert wird.

15. Verfahren nach einem der Ansprüche 9 bis 14, **dadurch gekennzeichnet, dass** Verbindung (III) in Schritt (C) durch Basenzusatz, vorzugsweise durch Zusatz von NaOH, aus dem Salz freigesetzt wird.

16. Verfahren nach Anspruch 15, **dadurch gekennzeichnet, dass** Schritt (C) unter Erhitzen durchgeführt wird, wobei die aus dem Salz freigesetzte Verbindung (III) in geschmolzener Form erhalten wird.

17. Verfahren nach Anspruch 16, **dadurch gekennzeichnet, dass** die in geschmolzener Form vorliegende Verbindung (III) abgekühlt und nach dem Erstarren aus wässriger alkalischer Lösung kristallisiert wird.

18. Verfahren zur Herstellung von Verbindung (III), ausgehend von einem Salz dieser Verbindung, **dadurch gekennzeichnet, dass** Verbindung (III) als freie Base in geschmolzener Form aus dem Salz freigesetzt und isoliert wird.

19. Verfahren nach Anspruch 18, **dadurch gekennzeichnet, dass** die in geschmolzener Form vorliegende Verbindung (III) abgekühlt und nach dem Erstarren aus wässriger alkalischer Lösung kristallisiert wird.

20. Verfahren zur Herstellung einer Verbindung der angegebenen Formel (III), **dadurch gekennzeichnet, dass** es einen Schritt aufweist, bei welchem diese Verbindung in flüssiger Form aus dem Reaktionsansatz abgeschieden wird.

21. Verfahren zur Herstellung einer Verbindung der angegebenen Formel (III) nach Anspruch 20, **dadurch gekennzeichnet, dass** es folgende Schritte umfasst:
- Reduktion der genannten Verbindung (I) zu Verbindung (III), wobei Verbindung (III) in Salzform anfällt;
- Zusatz einer Base, wodurch Verbindung (III) aus dem Salz freigesetzt und in flüssiger Form abgeschieden wird.

22. Verwendung einer Verbindung der Formel (I), hergestellt nach einem Verfahren nach einem der Ansprüche 1 bis 9, zur Herstellung einer Verbindung der Formel (III) als freie Base oder in Form eine Salzes.

## Claims

1. Process for the production of a compound of the following formula (I) 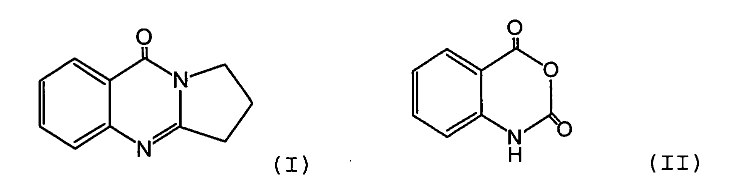 by converting a compound of formula (II) with 2-pyrrolidone, **characterized in that** an excess of 2-pyrrolidone is used, relative to compound (II),

2. Process according to claim 1, **characterized in that** in a subsequent step the reaction product (I) is isolated directly from the reaction mixture by crystallisation.

3. Process according to claim 1 or 2, **characterized in that** 1.5 to 5 mol, preferably 2 to 4 mol, especially preferably 2.5 to 3.5 mol, of 2-pyrrolidone are used, each value relative to the amount of compound (II).

4. Process according to any one of the preceding claims, **characterized in that** the conversion is performed at 50 to 200 °C.

5. Process according to claim 4, **characterized in that** the temperature of the reaction mixture is initially heated to 70 to 130 °C, preferably to 80 to 120 °C, and subsequently to 140 to 200 °C, preferably 150 to 190 °C.

6. Process according to claim 5, **characterized in that** the said initial temperature is maintained for a period of 0.5 to 2 h, preferably 1 to 1.5 h, and the said subsequent temperature is maintained for a period of 1 to 8 h, preferably from 2 to 5 h.

7. Process according to claim 6, **characterized in that** the reaction mixture is, after cooling, seeded with seed crystals of compound (I), and is maintained at room temperature, preferably at least 25 °C, for a period of 24 h to 7 days, preferably 50 to 100 h, to enable crystallisation.

8. Process according to claim 7, **characterized in that** the crystallisation is carried through at 30 to 70 °C, preferably at 40 to 60 °C.

9. Process for the production of a compound of the following formula (III), comprising the following steps: 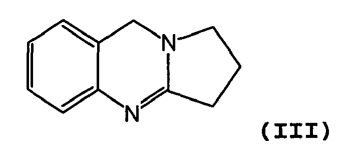
(A) Preparing compound (I) according to a process according to any one of claims 1 to 8;
(B) reduction reaction, giving compound (III) in salt form;
(C) liberating compound (III) from the salt.

10. Process according to claim 9, **characterized in that** a reaction product isolated by crystallisation according to any one of claims 1 to 8 is used as compound (I).

11. Process according to claim 9 or 10, **characterized in that** the reduction reaction (step B) is performed in the presence of zinc and acid.

12. Process according to claim 11, **characterized in that** compound (I), preferably in crystallised form, is initially dissolved in glacial acetic acid and that subsequently zinc and hydrochloric acid are added.

13. Process according to claim 12, **characterized in that** the reduction reaction is performed in the presence of aqueous sulfuric acid and zinc dust.

14. Process according to any one of claims 9 to 13, **characterized in that** subsequent to step (B), compound (III) is isolated as a salt by crystallisation from the reaction mixture.

15. Process according to any one of claims 9 to 14, **characterized in that** in step (C) the compound (III) is liberated from the salt by addition of a base, preferably by addition of NaOH.

16. Process according to claim 15, **characterized in that** step (C) is carried through under heating, with the compound (III), which is liberated from the salt, being obtained in molten form.

17. Process according to claim 16, **characterized in that** the compound (III) present in molten form is cooled down and, after freezing, is crystallised from aqueous alkaline solution.

18. Process for the production of compound (III), starting from a salt of said compound, **characterized in that** compound (III) is liberated and isolated from the salt as a free base in molten form.

19. Process according to claim 18, **characterized in that** the compound (III) present in molten form is cooled down and, after freezing, is crystallised from aqueous alkaline solution.

20. Process for the production of a compound of formula (III) as indicated, **characterized in that** it contains a step wherein this compound is separated from the reaction mixture in liquid form.

21. Process for the production of a compound of formula (III) as indicated, according to claim 20, **characterized in that** said process comprises the following steps:
- reducing the aforementioned compound (I) to compound (III), which gives compound (III) in salt form;
- adding a base, whereby compound (III) is liberated from the salt and separates out in liquid form.

22. Use of a compound of formula (I), produced according to a process according to any one of claims 1 to 9, for the production of a compound of formula (III) as a free base or in the form of a salt.

## Revendications

1. Procédé pour la préparation d'un composé présentant la formule suivante (I) 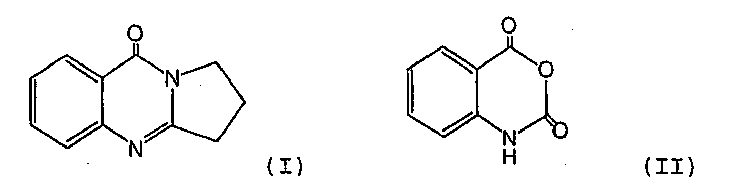 par transformation d'un composé de formule (II) avec de la 2-pyrrolidone, **caractérisé en ce que** la 2-pyrrolidone est utilisée en excès par rapport au composé (II).

2. Procédé selon la revendication 1, **caractérisé en ce que** le produit de réaction (I) est isolé dans une étape consécutive directement à partir du mélange réactionnel par cristallisation.

3. Procédé selon la revendication 1 ou 2, **caractérisé en ce** 1,5 à 5 moles, de préférence 2 à 4 moles, de manière particulièrement préférée 2,5 à 3,5 moles de 2-pyrrolidone sont utilisées, à chaque fois par rapport à la quantité de composé (II).

4. Procédé selon l'une quelconque des revendications précédentes, **caractérisé en ce que** la transformation est réalisée à 50 jusqu'à 200°C.

5. Procédé selon la revendication 4, **caractérisé en ce que** le mélange réactionnel est équilibré thermiquement, au départ à 70 jusqu'à 130°C, de préférence à 80 jusqu'à 120°C, et ensuite à 140 jusqu'à 200°C, de préférence à 150 jusqu'à 190°C.

6. Procédé selon la revendication 5, **caractérisé en ce que** la température de départ mentionnée est maintenue sur un laps de temps de 0,5 à 2 h, de préférence de 1 à 1,5 h, et la température consécutive mentionnée est maintenue sur un laps de temps de 1 à 8 h, de préférence de 2 à 5 h.

7. Procédé selon la revendication 6, **caractérisé en ce que** la charge réactionnelle est ensemencée après le refroidissement avec des cristaux d'ensemencement du composé (I) et est conservée sur un laps de temps de 24 h à 7 jours, de préférence de 50 à 100 h, à température ambiante, de préférence à au moins 25°C, pour permettre la recristallisation.

8. Procédé selon la revendication 7, **caractérisé en ce que** la cristallisation est réalisée à 30 jusqu'à 70°C, de préférence à 40 jusqu'à 60°C.

9. Procédé pour la préparation d'un composé présentant la formule suivante (III), comprenant les étapes suivantes: 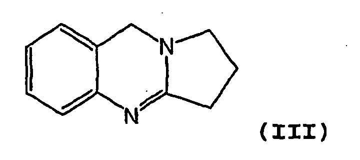
(A) préparation du composé (I) selon un procédé selon l'une quelconque des revendications 1 à 8 ;
(B) réaction de réduction, le composé (III) étant obtenu sous forme de sel ;
(C) libération du composé (III) à partir du sel.

10. Procédé selon la revendication 9, **caractérisé en ce qu'**on utilise comme composé (I) un produit de réaction selon l'une quelconque des revendications 1 à 8 isolé par cristallisation.

11. Procédé selon la revendication 9 ou 10, **caractérisé en ce que** la réaction de réduction (étape B) est réalisée en présence de zinc et d'acide.

12. Procédé selon la revendication 11, **caractérisé en ce que** le composé (I), de préférence sous forme cristallisée, est d'abord dissous dans de l'acide acétique glacial, puis on ajoute du zinc et de l'acide chlorhydrique.

13. Procédé selon la revendication 12, **caractérisé en ce que** la réaction de réduction est réalisée en présence d'acide sulfurique aqueux et de poussière de zinc.

14. Procédé selon l'une quelconque des revendications 9 à 13, **caractérisé en ce que** le composé (III) est isolé après l'étape (B) sous forme de sel par cristallisation à partir de la charge réactionnelle.

15. Procédé selon l'une quelconque des revendications 9 à 14, **caractérisé en ce que** le composé (III) dans l'étape (C) est libéré à partir du sel par addition de base, de préférence par addition de NaOH.

16. Procédé selon la revendication 15, **caractérisé en ce que** l'étape (C) est réalisée en chauffant, le composé (III) libéré à partir du sel étant obtenu sous forme fondue.

17. Procédé selon la revendication 16, **caractérisé en ce que** le composé (III) se trouvant sous forme fondue est refroidi et cristallisé après la solidification à partir d'une solution aqueuse alcaline.

18. Procédé pour la préparation du composé (III), partant d'un sel de ce composé, **caractérisé en ce que** le composé (III) est libéré sous forme de base libre sous forme fondue à partir du sel et est isolé.

19. Procédé selon la revendication 18, **caractérisé en ce que** le composé (III) se trouvant sous forme fondue est refroidi et cristallisé après la solidification à partir d'une solution aqueuse alcaline.

20. Procédé pour la préparation d'un composé présentant la formule indiquée (III), **caractérisé en ce qu'**il présente une étape dans laquelle ce composé est séparé sous forme liquide de la charge réactionnelle.

21. Procédé pour la préparation d'un composé présentant la formule indiquée (III), selon la revendication 20, **caractérisé en ce qu'**il comprend les étapes suivantes :
- réduction du composé mentionné (I) en composé (III), le composé (III) étant obtenu sous forme de sel ;
- addition d'une base, le composé (III) étant libéré à partir du sel et séparé sous forme liquide.

22. Utilisation d'un composé de formule (I), préparé selon un procédé selon l'une quelconque des revendications 1 à 9, pour la préparation d'un composé de formule (III) sous forme de base libre ou sous forme d'un sel.
